# EUROPEAN PATENT APPLICATION

(11) **EP 1 974 753 A1**
(43) Date of publication of application: **01.10.2008**
(21) Application number: 07105088.4
(22) Date of filing: 28.03.2007
(51) Int. Cl.: A61L 2/08, A61L 2/26, G21K 5/04, A47L 11/34

(54) **Portable electron beam sterilisation device**

(71) Applicant: Technology from Ideas, Dublin 12 (IE)
(72) Inventor: McEvoy, Paul, Dublin (IE)
(74) Representative: Lane, Cathal Michael

(57) **Abstract**

The present invention relates to a portable electron beam sterilisation device (1) comprising a sterilisation portion (2) which includes a housing (3), having a surface targeting portion (4). The sterilisation portion further includes an electron beam source (6) arranged within the housing and having an outlet (7) for emission of an electron beam through the surface targeting portion. The device also comprises a high voltage power unit (8), for converting mains supply to high voltage and supplying power to the electron beam source. The device comprises a connector for connection to a standard mains supply (13).

## Description

### Field of the Invention

The present invention relates to devices for sterilisation using electron beams, and in particular, to portable, single-user operable devices for sterilisation of surfaces using electron beams.

### Background to the Invention

Sterilisation of a surface is the elimination of all transmissible agents from that surface. Disinfection involves deactivation of organisms that can cause disease from a non-living object by application of an antimicrobial agent (or disinfectant) thereto. High-level disinfectants known as sanitisers can kill over 99.9% of a target microorganism in applicable situations. Not all disinfectants and sanitisers can sterilise, and those that can depend entirely on their mode of application. Certain microorganisms such as bacterial endospores, as well as some viruses and bacteria, are resistant to disinfectants.

In order to prevent transmission of disease and infection in hospitals, it is essential that surfaces are maintained free, or substantially free of microorganisms. In particular, with the increasing prevalence of antibiotic-resistant "superbugs" such as methicillin-resistant *Staphylococcus aureus* (MRSA), Salmonella or Clostridium Difficile (C-Diff) in hospitals, it is vital that transmission of infection is prevented.

Traditionally, surfaces such as floors and walls in hospitals and other clinical settings have been disinfected using liquid disinfectants such as hydrogen peroxide solution. Liquid disinfectants are quick and easy to use and are capable of eliminating most disease-causing bacteria. There are, however, a number of drawbacks to this approach. Liquid disinfectants can cause allergic reactions, particularly in sensitive persons, or those with respiratory problems. Furthermore, as discussed above, the result achieved is disinfection, rather than complete sterilisation and some microorganisms may be resistant to particular disinfectants. Additionally, liquid disinfectants may only be used on hard or waterproof surfaces such as floors and walls and may not be suitable for sterilisation of soft furnishings such as mattresses, curtains and chairs etc. An alternative approach to sterilisation is to seal a room and fill it with a hydrogen peroxide vapour. Such an approach gives very good disinfection but requires extensive preparation, the room has to be taken out of action for a significant period of time, and is not suitable for a wide range of environments.

Both the food and agriculture industries also have a need for portable sterilisation capability, to maintain high standards of hygiene in food production and packaging. The security and health and safety sectors also have a requirement for portable sterilisation to decontaminate areas after biological accidents.

Several methods of sterilisation using radiation are also known, for example, X-ray sterilisation, gamma ray sterilisation and ultra-violet sterilisation. However, these methods also have disadvantages associated with them. For example, gamma rays are very penetrating, and as a result require bulky shielding for the safety of the operators of the gamma irradiation apparatus. Furthermore, gamma ray sterilisation also requires storage of a radioisotope which continuously emits gamma rays (it cannot be turned off, and therefore always presents a hazard in the area of the facility). Although X-rays are less penetrating than gamma rays, they also require significant shielding to protect individuals from radiation exposure. This results in apparatus of significant weight which is unsuitable for portable sterilisation, e.g. of hospital rooms. Germicidal UV lamps have a much lower energy than X-rays and therefore have no significant penetration, making them suitable only for use on surfaces and some transparent objects. They also require a much longer exposure time than X-rays. UV is ineffective in shaded areas, including areas under dirt. It also damages many plastics.

It is therefore desirable to provide a means of sterilisation suitable for sterilising a variety of surfaces without the disadvantages associated with the methods described above.

Electron beams may also be used for sterilisation. Electron beams (e-beams) kill microbes by destroying vital molecules or causing a chemical reaction within the microorganism. They also impair the ability of bacteria to reproduce. They can damage DNA and RNA viruses through either direct effect on the nucleic acid or indirectly due to OH radicals originating from water present in the cell. Unlike most chemicals, they can also target both gram positive and gram negative bacteria. E-beam sterilisation does not lead to drug resistant bacteria and leaves no toxic residue.

Recent developments in electron beam accelerator technology have led to the development of compact modular KeV accelerators. These devices produce lower levels of X-ray radiation than earlier, larger accelerators and therefore require less shielding to protect the user.

The object of the invention is to provide a portable electron beam sterilisation device suitable for sterilisation of surfaces.

### Summary of the Invention

The present invention relates to a portable electron beam sterilisation device comprising a sterilisation portion, the sterilisation portion comprising a housing, having a surface targeting portion, and an electron beam source arranged within the housing and having an outlet for emission of an electron beam through the surface targeting portion, and the device further comprising a high voltage power unit, for converting mains supply to high voltage and supplying power to the electron beam source; and a connector for connection to a standard mains supply.

The term "mains" refers to the general purpose alternating current (AC) electrical power supply, also referred to as household power, household electricity, domestic power, wall power, line power, AC power, grid power or "hydro". Suitably, the connector is a standard household electrical plug engageable with a standard household electrical socket to connect the device to a 100 to 240 volt single-phase electrical supply.

The device may be operated by a single user. Preferably, the device is portable by a single user. This allows it to be easily moved and manoeuvred by one operator in use. The device may be adapted to be pushed, pulled or carried easily by a single person. Alternatively, the device may be a "ride-on" type device powered for movement by a user over a surface to be sterilised.

According to a first aspect, the invention relates to an electron beam sterilisation device comprising a sterilisation portion, which includes a housing, having a surface targeting portion, and an electron beam source arranged within the housing and having an outlet for emission of an electron beam having an energy between 0.1 and 0.5 MeV through the surface targeting portion; and a high voltage power unit, for converting mains supply to high voltage and supplying between 100 watts and 3000 watts of power to the electron beam source; and a connector for connection to a standard mains supply.

To achieve a required level of sterilisation, a specific dosage must be delivered to the surface. The current and voltage requirements are selected to deliver the desired dosage to a desired depth across a particular surface area within a suitable period of time. A dosage of 10kGy with a penetration depth of about 200 microns at a cleaning speed of 2000 square centimetres per second (i.e. 1 square metre in 5 seconds) would be suitable for most applications. Careful selection of operating parameters ensures that the power requirements of the device may be fulfilled by a single-phase supply, that is, by connection to a conventional wall socket.

According to a second aspect of the invention, the sterilisation portion and the power unit are arranged separately and the device further comprises a cable arranged therebetween for transfer of power to the electron beam source. The cable may be a shielded high voltage power cable.

An advantage of this arrangement is that the power unit, which may be relatively heavy, is allowed to rest in one place and may be wheeled around if necessary, while the portion of the device used for sterilisation may be kept relatively light and thus easier to manoeuvre.

According to one embodiment, the sterilisation portion may be adapted to be carried by a user. This allows the sterilisation portion to be aimed at any surface or area, thereby allowing non-uniform or vertical surfaces, such as walls and curtains, to be sterilised easily. The energy of the electron beam defines the distance from which the surfaces may be sterilised, which is preferably in the region of 1 metre.

According to another aspect of the invention, there is provided a portable electron beam sterilisation device comprising a sterilisation portion, which includes a housing, having a surface targeting portion, and an electron beam source arranged within the housing and having an outlet for emission of an electron beam through the surface targeting portion; and a handle, arranged to allow the sterilisation portion to be scanned over a surface to be sterilised. Preferably the handle is dimensioned to prevent a user from accidentally irradiating himself. For example, the handle may be at least one metre in length to prevent the user from accidentally directing the electron beam at himself.

Alternatively, the device comprises a harness or other attachment means to allow the sterilisation portion to be worn or carried by a user. The harness or attachment means is adapted so that, in use, the surface targeting portion of the device is directed away from the user, to prevent the user from accidentally irradiating himself.

According to another aspect of the invention, the device comprises a vacuum cleaning system. The sterilisation portion may comprise a vacuum cleaning head at a lower end thereof, such that the surface may be vacuum cleaned and sterilised simultaneously.

This particular arrangement has two advantages. The first advantage is that the surface can be cleaned and sterilised at the same time. This means that it is not necessary to vacuum clean a floor to remove dust and debris before or after sterilisation, as the two operations are performed simultaneously or sequentially.

The vacuum suction also has the further advantage of removing any gases that may be formed by the interaction of the electron beam with the surface.

The device may comprise a surface washing system for applying a cleaning solution to the surface. The sterilisation portion may comprise a nozzle for directing a cleaning solution at the surface to be washed and a suction system for drawing the cleaning solution back into the device. This allows the surface to be washed and sterilised simultaneously, or the device may allow the washing and sterilisation to be carried out sequentially. The device may include both the vacuum cleaning system and the washing system. In one embodiment the device is arranged to allow a surface to be vacuumed first, then washed and then sterilised.

According to a further aspect of the invention, the device comprises a pumping system, wherein the sterilisation portion comprises a gas pump operable to pump gas from the device into a region between the sterilisation portion and a surface to be sterilised. This allows any gases formed by interaction of the electron beam with the surface to be blown away. The gas used may be, for example, air or nitrogen. Nitrogen has the advantage of enhancing the stability of the electron beam.

According to yet another aspect of the invention, the device comprises a sensor for sensing proximity of the sterilisation portion of the device to a surface to be sterilised; and means for preventing operation of the electron beam source if the distance between the sterilisation portion and the surface exceeds a threshold. The sensor may be arranged at or adjacent to the surface targeting portion to sense the proximity of the surface targeting portion to the surface to be sterilised. The sensor may be arranged to detect a change in proximity of the surface targeting portion to the surface to be sterilised. Suitably, the sensor is arranged to detect an increase in the distance between the surface targeting portion and the surface to be sterilised.

According to another aspect of the invention, the electron beam sterilisation device comprises a sensor for sensing proximity of the device to other objects and means for preventing operation of the electron beam source if the distance between the sterilisation portion and an object is below a threshold. Such sensors may be adapted to detect only non-static objects or objects with specific heat signatures, such as humans. The sensor may be arranged at or adjacent the surface targeting portion to sense the proximity of the sterilisation portion to other objects. The sensor may be arranged to detect a change in proximity of the sterilisation portion to an object. Suitable, the sensor is arranged to detect a decrease in the distance between the sterilisation portion and an object.

This allows the electron beam source to be shut off if an object, the user or another person gets too close to the surface targeting portion of the device and thereby prevents accidental irradiation of the user or other persons or objects in the vicinity of the device.

The sensor may be an infrared detection (for detection of body heat), a proximity sensor or a motion detector.

The device may further comprise a control system for controlling the energy of the electron beam emitted by the source. The control system may be arranged to reduce the energy of the electron beam when the sterilisation portion is moved slowly and to increase the energy of the electron beam when the sterilisation portion is moved more quickly, thereby ensuring a consistent dose over the entire surface to be sterilised. The system may also be arranged to shut off power to the source if the sterilisation portion is stationary. The control system may comprise a motion sensor (for example, an accelerometer), a camera, or an image recognition system to detect motion of the device and adjust the energy of the beam accordingly.

According to another aspect of the invention, the electron beam sterilisation device comprises a guidance system operable to indicate to a user the area being sterilised. The guidance system may comprise a plurality of lights arranged to illuminate a portion of a surface being sterilised. Alternatively, the guidance system may comprise a physical frame or structure mounted on the device to indicate the borders of an area being sterilised.

The guidance system may also comprise means for indicating whether the device is being operated within an optimal range from a surface. The means for indicating may comprise a second plurality of lights arranged to emit a beam of light at an angle to that emitted by the first plurality of lights. If the device is operated within the optimal range from the surface, the beam emitted by the second plurality of lights will be visible on the surface on one side of the beam emitted by the first plurality of lights. However, if the device is operated outside the optimal range, the beams emitted by the second plurality of lights will be visible on the surface on the other side of the beam emitted by the first plurality of lights. The second set of lights may emit a different colour light than that emitted by the first set of lights.

Alternatively, the means for indicating may comprise a sensor for measuring the distance to the surface and a means for providing a warning to the user if the optimal range is exceeded.

Long-term exposure to low-level X-ray radiation may have certain health implications. Careful choice of operating conditions and device design ensures that the level of X-ray radiation generated by electron beam sterilisation will be low or negligible, and the sterilisation portion housing may be sufficient to absorb any generated X-rays, thereby preventing X-ray radiation from reaching the user. However, the device may further comprise shielding arranged to prevent user irradiation by X-rays produced by the electron beam source.

High-density metal targets may generate X-rays if irradiated with an electron beam of sufficient energy. The sterilisation portion may comprise shielding arranged to prevent backscatter of surface emitted X-rays produced by interaction of the electron beam and a surface to be sterilised. The thickness of the shielding may be chosen depending on the material from which it is formed. High-density materials will require lower thicknesses to provide adequate shielding.

While certain features of the invention are, for clarity, described in the context of separate embodiments or aspects of the invention, these features may also be provided in combination in a single embodiment. Conversely, various features of the invention which are, for brevity, described in the context of a single embodiment or aspect of the invention, may also be provided separately or in any suitable sub-combination.

### Brief Description of the Drawings

Figure 1 is a schematic view of a first embodiment of the electron beam sterilisation device according to the present invention;
Figure 2 is a schematic view of a second embodiment of the electron beam sterilisation device of the present invention; and
Figure 3 is a schematic view of a third embodiment of the electron beam sterilisation device of the present invention.

### Detailed Description of the Drawings

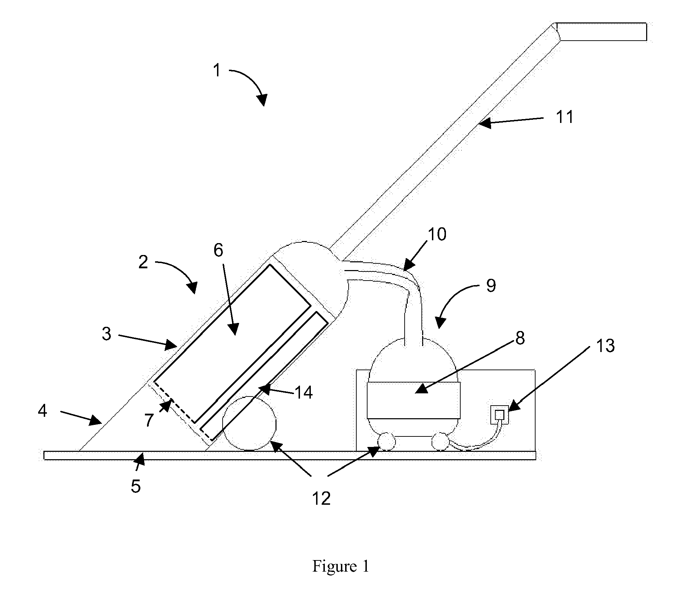

An embodiment of the electron beam sterilisation device 1 according to the present invention is shown in Figure 1. The device 1 comprises a sterilisation portion or head 2. The sterilisation portion 2 includes a housing 3, which has a surface targeting portion 4 at a lower end 5 thereof. The sterilisation portion 2 further comprises an electron beam accelerator 6 arranged within the housing 3 and having an outlet 7 for emission of an electron beam having an energy between 0.1 and 0.5 MeV through the surface targeting portion 4. The beam emitted is approximately 20cm long and 10cm wide. In use, the housing 3 shields the operator and the surrounding environment from any radiation generated by the electron beam source.

The device 1 further comprises a high voltage power unit 8, for supplying power to the electron beam source 6. The power required to produce the sterilisation dose is such that the device may be powered by connection to a single-phase supply, 13.

In the embodiment shown in Figure 1, sterilisation portion 2 and power unit 8 are arranged separately, in a similar manner to a cylinder-type vacuum cleaner. The power unit 8 is provided with its own separate housing 9 and a shielded high voltage power cable 10 is arranged between the power unit 8 and the sterilisation portion 2 for transfer of power to the electron beam source 6. A mains to high voltage converter and control electronics are also provided within the housing 9. The device 1 further comprises a handle or wand 11 to allow the head 2 to be scanned over a surface to be sterilised, in a similar manner to vacuum cleaning. In the embodiment shown, the handle 11 is approximately one metre in length. This prevents the user from accidentally directing the electron beam at himself. The length of the handle or wand may be adjustable to allow taller users to use the device comfortably. Preferably, the minimum wand length is one metre, to prevent the user from accidentally directing the electron beam at himself.

The device 1 may further comprise a vacuum cleaning system 14, wherein the sterilisation portion 2 comprises a vacuum cleaning head at a lower end thereof, whereby the surface may be vacuum cleaned and sterilised simultaneously. The power unit housing 9 and the sterilisation portion 2 are each provided with wheels 12 to allow them to be moved around as necessary. This allows the device to be easily operated by a single user.

In the embodiment shown in Figure 1, the device 1 further comprises sensors in the head 2 of the device used to sense the proximity of objects surrounding the head. The sensors are connected to a control system, which is arranged to shut off power to the electron beam accelerator 6 when the head 2 is too close to a nearby object, such as a person. The control system may also be arranged to sense the speed of movement of the device and the distance of the surface engaging portion from the surface and adjust the power accordingly. If the system senses that the surface engaging portion is no longer in close proximity to a surface, it will shut off power to the accelerator 6. Similarly, if the system senses that that the speed of movement of the device has reduced, it will reduce the power to avoid applying an excessive electron beam dose to one area of the surface. If the control system senses that the device has stopped moving completely, power to the accelerator will be shut off.

In the embodiment shown in Figure 1, the housing 3 of the sterilisation portion comprises a metallic material. Because of the low electron beam dose emitted from the accelerator 6, this is sufficient to shield the user from any backscattered X-rays created when the electron beam is directed at metal objects. In alternative embodiment, the housing may comprise further shielding.

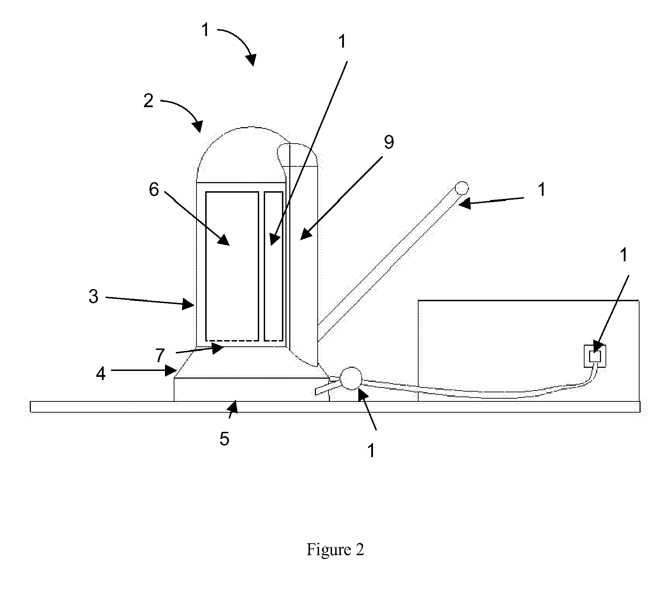

A second embodiment of the electron beam sterilisation device 1 according to the present invention is shown in Figure 2. The device 1 comprises a sterilisation portion or head 2. The sterilisation portion 2 includes a housing 3, which has a surface engaging portion 4 at a lower end 5 thereof. The sterilisation portion 2 further comprises an electron beam accelerator 6 arranged within the housing 3 and having an outlet 7 for emission of an electron beam having an energy between 0.1 and 0.5 MeV through the surface engaging portion 4. The exposure area is approximately 20cm long and 10cm wide.

The device 1 further comprises a power unit 9, for supplying power to the electron beam source 6. The power required to produce the sterilisation dose is such that the device may be powered by connection to a single-phase supply.

In the embodiment shown in Figure 2, power unit 9 is directly connected to sterilisation portion 2, in a similar manner to a floor polisher. The power unit 9 and sterilisation portion 2 are provided within a single housing 3. The device 1 further comprises a handle 11 to allow the sterilisation portion 2 to be scanned over a surface to be sterilised, in a similar manner to floor polishing. Because of the additional weight of the power unit, the user is prevented from accidentally lifting the sterilisation portion from the floor being sterilised. This prevents the user from accidentally directing the electron beam at himself.

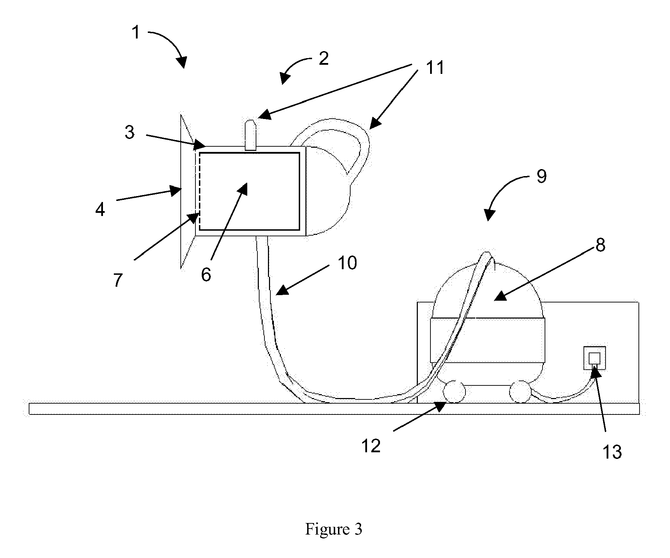

A third embodiment of the electron beam sterilisation device according to the present invention is shown in Figure 3. The device 1 comprises a sterilisation portion or head 2, which includes a housing 3. The housing 3 has a surface targeting portion 4. The sterilisation portion 2 further comprises an electron beam accelerator 6 arranged within the housing 3 and having an outlet 7 for an electron beam through the targeting portion 4. The exposure area is approximately 20cm long and 10cm wide.

The device 1 further comprises a power unit 9 for supplying power to the electron beam source. The power required to produce the sterilisation dose is such that the device may be powered by connection to a single-phase supply 13.

In the embodiment of Figure 3, the power unit 9 is connected to the sterilisation portion 2 by means of a shielded high voltage power cable 10. The sterilisation unit 2 further comprises handles 11 to allow the sterilisation portion 2 to be carried and aimed at any surface or object to be sterilised. Such objects would ideally be within 10cm of the targeting portion 4, with the maximum operating range determined by the energy of the electron beam. The sterilisation portion 2 further comprises sensors to detect the presence of people or unsuitable materials for sterilisation within the range of the electron beam, and means to prevent operation of the electron beam source 6 if such are detected. The sterilisation portion 2 is designed such that it requires the user to operate it with both hands and is shaped to make it impossible for the targeting portion 4 to be aimed back at the user, thereby preventing the user from accidentally irradiating himself.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A portable electron beam sterilisation device comprising:
a sterilisation portion, comprising:
a housing, having a surface targeting portion; and
an electron beam source arranged within the housing and having an outlet for emission of an electron beam through the surface targeting portion; and
a high voltage power unit, for converting mains supply to high voltage and supplying power to the electron beam source; and
a connector for connection to a standard mains supply.

2. A portable electron beam sterilisation device comprising:
a sterilisation portion, comprising:
a housing, having a surface targeting portion; and
an electron beam source arranged within the housing and having an outlet for emission of an electron beam through the surface targeting portion; and
a vacuum cleaning system, wherein the sterilisation portion comprises a vacuum cleaning head adjacent the surface targeting portion, such that the surface may be vacuum cleaned and sterilised simultaneously or sequentially.

3. A portable electron beam sterilisation device comprising:
a sterilisation portion, comprising:
a housing, having a surface targeting portion; and
an electron beam source arranged within the housing and having an outlet for emission of an electron beam through the surface targeting portion; and
a surface washing system for applying a cleaning solution to the surface, such that the surface may be washed and sterilised simultaneously or sequentially.

4. A portable electron beam sterilisation device as claimed in claim 3, wherein the sterilisation portion comprises a nozzle for applying the cleaning solution to the surface and a suction system for drawing the cleaning solution back into the device.

5. A portable electron beam sterilisation device comprising:
a sterilisation portion, comprising:
a housing, having a surface targeting portion; and
an electron beam source arranged within the housing and having an outlet for emission of an electron beam having an energy between 0.1 and 0.5 MeV through the surface targeting portion;
and
a high voltage power unit, for converting mains supply to high voltage and supplying between 100 watts and 3000 watts of power to the electron beam source; and
a connector for connection to a standard mains supply.

6. An electron beam sterilisation device as claimed in claim 5, wherein the high voltage power unit is for supplying between 500 and 1500 watts of power to the electron beam source.

7. A portable electron beam sterilisation device comprising:
a sterilisation portion, comprising:
a housing, having a surface targeting portion; and
an electron beam source arranged within the housing and having an outlet for emission of an electron beam through the surface targeting portion; and
a sensor for sensing proximity of the sterilisation portion of the device to a surface to be sterilised; and
means for preventing operation of the electron beam source if the distance between the sterilisation portion and the surface exceeds a threshold.

8. An electron beam sterilisation device as claimed in claim 7, wherein the sensor is arranged at or adjacent to the surface targeting portion to sense the proximity of the surface targeting portion to the surface to be sterilised.

9. A portable electron beam sterilisation device comprising:
a sterilisation portion, comprising:
a housing, having a surface targeting portion; and
an electron beam source arranged within the housing and having an outlet for emission of an electron beam through the surface targeting portion; and
a sensor for sensing proximity of the device to other objects; and
means for preventing operation of the electron beam source if the distance between the sterilisation portion and an object is below a threshold.

10. An electron beam sterilisation device as claimed in claim 9, wherein the sensor is arranged at or adjacent the surface targeting portion to sense the proximity of the sterilisation portion to other objects.

11. A portable electron beam sterilisation device comprising:
a sterilisation portion, comprising:
a housing, having a surface targeting portion; and
an electron beam source arranged within the housing and having an outlet for emission of an electron beam through the surface targeting portion; and
a high voltage power unit, for converting mains supply to high voltage and supplying power to the electron beam source; and
a connector for connection to a standard mains supply;
wherein the sterilisation portion and the power unit are arranged separately and further comprising a cable arranged therebetween for transfer of power to the electron beam source.

12. An electron beam sterilisation device as claimed in claim 11, wherein the cable is a high voltage shielded power cable.

13. A portable electron beam sterilisation device comprising:
a sterilisation portion, comprising:
a housing, having a surface targeting portion; and
an electron beam source arranged within the housing and having an outlet for emission of an electron beam through the surface targeting portion; and
a handle, arranged to allow the sterilisation portion to be scanned over a surface to be sterilised.

14. A portable electron beam sterilisation device as claimed in claim 13, wherein the handle is dimensioned to prevent a user from accidentally irradiating himself.

15. A portable electron beam sterilisation device comprising:
a sterilisation portion, comprising:
a housing, having a surface targeting portion; and
an electron beam source arranged within the housing and having an outlet for emission of an electron beam through the surface targeting portion; and
a harness, arranged to allow the sterilisation portion to be worn or carried by a user and scanned over a surface to be sterilised.

16. A portable electron beam sterilisation device comprising:
a sterilisation portion, comprising:
a housing, having a surface targeting portion; and
an electron beam source arranged within the housing and having an outlet for emission of an electron beam through the surface targeting portion; and
a pumping system, wherein the sterilisation portion comprises a gas pump operable to pump gas from the device into a region between the sterilisation portion and a surface to be sterilised.

17. An electron beam sterilisation device as claimed in claim 16, wherein the gas is nitrogen.

18. A portable electron beam sterilisation device comprising:
a sterilisation portion, comprising:
a housing, having a surface targeting portion; and
an electron beam source arranged within the housing and having an outlet for emission of an electron beam through the surface targeting portion; and
a control system for controlling the electron beam dose emitted by the source.

19. A portable electron beam sterilisation device comprising:
a sterilisation portion, comprising:
a housing, having a surface targeting portion; and
an electron beam source arranged within the housing and having an outlet for emission of an electron beam through the surface targeting portion;
a guidance system operable to indicate to a user the area being sterilised.

20. A portable electron beam sterilisation device comprising:
a sterilisation portion, comprising:
a housing, having a surface targeting portion;
an electron beam source arranged within the housing and having an outlet for emission of an electron beam through the surface targeting portion; and
shielding arranged to prevent user irradiation by X-rays produced by the electron beam source.

21. A portable electron beam sterilisation device comprising:
a sterilisation portion, comprising:
a housing, having a surface targeting portion;
an electron beam source arranged within the housing and having an outlet for emission of an electron beam through the surface targeting portion; and
shielding arranged to prevent backscatter of surface emitted X-rays produced by interaction of the electron beam and a surface to be sterilised.

22. A portable electron beam sterilisation device as claimed in any preceding claim, portable by a single operator.

23. A portable electron beam sterilisation device as claimed in any preceding claim, adapted to be pushed, pulled or carried by a single operator.

24. A portable electron beam sterilisation device as claimed in any preceding claim, powered for movement by a user.

25. An electron beam sterilisation device substantially as hereinbefore described with reference to and/or as illustrated in Figure 1, Figure 2 or Figure 3 of the accompanying drawings.
